# EUROPÄISCHE PATENTSCHRIFT

(11) **EP 3 166 589 B1**
(45) Veröffentlichungstag und Bekanntmachung des Hinweises auf die Patenterteilung: **08.04.2020**
(21) Anmeldenummer: 15733722.1
(22) Anmeldetag: 01.07.2015
(51) Int. Cl.: A61K 8/64, A61Q 15/00, A61K 8/9789

(54) **SCHWEISSHEMMENDE KOSMETISCHE MITTEL MIT SPEZIELLEN PROTEINEN AUS HÜLSENFRÜCHTEN DER GATTUNG PISUM, WELCHE KEINE HALOGENIDE UND/ODER HYDROXYHALOGENIDE VON ALUMINIUM UND/ODER ZIRCONIUM ENTHALTEN"**
SWEAT-INHIBITING COSMETICS WITH PROTEINS FROM LEGUME OF GENUS PISUM, AND WHICH DO NOT CONTAIN ALUMINIUM AND/OR ZIRCONIUM HALOGENIDES AND/OR HYDROXYHALOGNIDES
COMPOSITIONS COSMETIQUES COMPRENANT DES PROTEINES DE LÉGUMINEUSES DU GENRE PISUM, ET QUI NE COMPRENNENT PAS DES HALOGÉNURES ET/OU HYDROXYHALOGÉNURES D'ALUMINIUM ET/OU DE ZIRCONIUM

(30) Priorität: 08.07.2014 DE 102014213227; 26.08.2014 DE 102014216908
(43) Veröffentlichungstag der Anmeldung: 17.05.2017
(73) Patentinhaber: Henkel AG & Co. KGaA, 40589 Düsseldorf (DE)
(72) Erfinder: BANOWSKI, Bernhard, 40597 Düsseldorf (DE); EVERS, Stefan, 42781 Haan (DE)
(86) Internationale Anmeldenummer: PCT/EP2015/064993
(87) Internationale Veröffentlichungsnummer: WO 2016/005243

(56) Entgegenhaltungen:
- EP-A- 2 143 418
- EP-A1- 0 532 465
- EP-A2- 1 813 310
- WO-A1-94/24993
- WO-A1-2010/003861
- WO-A1-2010/070143
- WO-A1-2011/128530
- FR-A1- 2 796 839
- FR-A1- 2 944 446
- FR-A1- 2 971 159
- KR-B1- 100 968 391
- DATABASE GNPD [Online] MINTEL; Januar 2014 (2014-01), "24H Anti-Perspirant Deodorant Spray", XP002744351, Database accession no. 2301648

## Beschreibung

Die vorliegende Erfindung betrifft die Verwendung einer Kombination, aus kosmetischen Ölen, Treibmittel sowie mindestens einem aus Hülsenfrüchten der Gattung *Pisum* isoliertem Protein zur Reduzierung und/oder Verhinderung von Schweiß, insbesondere Achselschweiß oder Schweiß anderer Körperregionen.

Weiterhin betrifft die vorliegende Erfindung die Verwendung mindestens eines speziellen Proteins zur zumindest teilweisen Beeinflussung der Schweißdrüse(n).

Das Waschen, Reinigen und Pflegen des eigenen Körpers stellt ein menschliches Grundbedürfnis dar und die moderne Industrie versucht fortlaufend, diesen Bedürfnissen des Menschen in vielfältiger Weise gerecht zu werden. Besonders wichtig für die tägliche Hygiene ist die anhaltende Beseitigung oder zumindest Reduzierung des Körpergeruchs und der Achselnässe. Im Stand der Technik sind zahlreiche spezielle deodorierende oder schweißhemmende Körperpflegemittel bekannt, welche für die Anwendung in Körperregionen mit einer hohen Dichte von Schweißdrüsen, insbesondere im Achselbereich, entwickelt wurden. Diese sind in den unterschiedlichsten Darreichungsformen konfektioniert, beispielsweise als Puder, in Stiftform, als Aerosolspray, Pumpspray, flüssige und gelförmige Roll-on-Applikation, Creme, Gel und als getränkte flexible Substrate (Deotücher).

Kosmetische Antitranspirantien des Standes der Technik enthalten neben mindestens einem Öl oder einem Wachs und einer Riechstoffkomponente bzw. einem Parfüm mindestens eine schweißhemmende Verbindung, insbesondere in Form von Halogeniden und/oder Hydroxyhalogeniden von Aluminium und/oder Zirconium. Diese schweißhemmenden Verbindungen verringern zum einen die Schweißsekretion des Körpers durch eine temporäre Verengung und/oder Verstopfung der Ausführungsgänge der Schweißdrüsen, so dass die Schweißmenge um etwa 20 bis 60 Prozent reduziert werden kann. Zum anderen weisen sie aufgrund ihrer antimikrobiellen Wirkung einen zusätzlichen desodorierenden Effekt auf.

Halogenide und/oder Hydroxyhalogenide von Aluminium und/oder Zirconium können in Verbindung mit dem sauren pH-Wert dieser Antitranspirantien bei einigen Anwendern zu unangenehmen Hautreaktionen führen. Darüber hinaus kann die Verwendung der vorgenannten schweißhemmenden Verbindungen zu einer Fleckenbildung auf der Kleidung führen.

Die FR 2 796 839 A1 und die KR 100 968 391 B1 offenbaren kosmetische Zusammensetzungen mit Vigna-Protein und kosmetischen Ölen.

Pepid-Extrakte aus *Pisum sativum* sowie ölhaltige Hautkosmetika enthaltend diese, werden in der WO 2011/28530 A1, der FR 2 791 159 A1 und der EP 2 143 418 A offenbart.

Auch die FR 2 944 446 A1 und die EP 0 532 465 A1 offenbaren Peptidextrakte und deren Einsatz in Hautkosmetika.

EP 1 813 310 A1 offenbart Stiftzusammensetzungen, welche neben Öl- und Wachskomponenten Sojaproteinhydrolysate enthalten.

WO 2010/003861 A1 beschreibt ein Kit, enthaltend zwei separate Zusammensetzungen, deren eine Petid enthält.

Es besteht daher ein Bedarf schweißhemmende Halogenide und/oder Hydroxyhalogenide von Aluminium und/oder Zirconium gegen andere schweißhemmende kosmetische Wirkstoffe auszutauschen. Diese schweißhemmenden Wirkstoffe sollen eine gute schweißhemmende Wirkung, eine gute Hautverträglichkeit sowie eine einfache Formulierbarkeit aufweisen. Darüber hinaus sollen diese schweißhemmenden Wirkstoffe keinen negativen Einfluss auf die Lagerstabilität der schweißhemmenden kosmetischen Mittel aufweisen.

Der vorliegenden Erfindung lag die Aufgabe zugrunde, ein schweißhemmendes kosmetisches Mittel bereitzustellen, welches die Nachteile des Standes der Technik vermeidet bzw. zumindest abschwächt und welches eine gute Hautverträglichkeit bei gleichzeitig zuverlässiger Verminderung der Achselnässe besitzt. Darüber hinaus soll das schweißhemmende kosmetische Mittel eine hohe Lagerstabilität aufweisen.

Es wurde nun überraschend gefunden, dass der Einsatz mindestens eines Proteins aus Hülsenfrüchten der Gattung *Pisum,* welches bei einer Änderung des pH-Werts von mindestens 0,5 in einem pH-Bereich von pH 4,0 bis pH 8,0 eine Veränderung der Lichtabsorption vom 1 bis 100 % bewirkt, in kosmetischen Mitteln ohne schweißhemmende Halogenide und/oder Hydroxyhalogenide von Aluminium und/oder Zirconium in einer schweißhemmenden Wirkung resultiert, welche mit der schweißhemmenden Wirkung von Formulierungen mit Aluminiumsalzen und/oder Aluminium-Zirkonium-Komplexen nahezu vergleichbar ist.

Gegenstand der vorliegenden Erfindung ist somit die Verwendung einer Kombination, enthaltend
a) mindestens einen Stoff, ausgewählt aus der Gruppe von kosmetischen Ölen, welche bei 20 °C und 1.013 hPa flüssig sind, Riechstoffen und Wachsen,
b) Treibmittel in einer Gesamtmenge von 0 bis 99 Gew.-%, bezogen auf das Gesamtgewicht des schweißhemmenden kosmetischen Mittels, und
c) mindestens ein Protein in einer Gesamtmenge von 0,1 bis 70 Gew.-%, bezogen auf das Gesamtgewicht des schweißhemmenden kosmetischen Mittels, wobei das mindestens eine Protein ein aus Hülsenfrüchten der Gattung *Pisum* isoliertes Protein ist, wobei das Protein hydrolysiert ist und gegebenenfalls kationisch modifiziert ist
und wobei das mindestens eine Protein bei einer pH-Wert Änderung von mindestens 0,5 in einem pH-Bereich von pH 4,0 bis pH 8,0, einer Temperatur von 20 °C bis 40 °C und einer Konzentration des Proteins von 0,001 bis 10 Gew.-%, bezogen auf das Gesamtgewicht der für die Bestimmung der Veränderung der Lichtabsorption verwendeten Probenmischung, eine Veränderung der Lichtabsorption von 1 bis 100 % - gemessen wie in der Beschreibung angegeben - bewirkt
und wobei die Kombination keine Halogenide und/oder Hydroxyhalogenide von Aluminium und/oder Zirconium enthält,
zur Reduzierung und/oder Verhinderung von Schweiß, insbesondere Achselschweiß oder Schweiß anderer Körperregionen.

Durch den Einsatz des mindestens einen Proteins aus Hülsenfrüchten der Gattung *Pisum* mit den zuvor genannten speziellen physikalischen Eigenschaften in der erfindungsgemäßen Verwendung erfolgt - ohne sich auf diese Theorie beschränken zu wollen - eine gezielte Beeinflussung der Schweißdrüse(n). Diese gezielte Beeinflussung der Schweißdrüse(n) kann beispielsweise in einer Gelbildung des mindestens einen Proteins bei pH-Werten, welche ausschließlich innerhalb der Ausführungsgänge der Schweißdrüsen vorliegen, bestehen. Auf diese Art und Weise kann eine effektive Verstopfung der Ausführungsgänge der Schweißdrüsen gewährleistet werden, ohne dass die schweißhemmende Wirkung des kosmetischen Mittels durch vorzeitige unerwünschte Gelbildung aufgrund des Zusatzes des mindestens einen speziellen Proteins vermindert wird. Weiterhin kann die gezielte die Beeinflussung der Schweißdrüse(n) jedoch auch in einer Störung des Ladungsgleichgewichts innerhalb der Schweißdrüse(n) bestehen, welche zu einer Beeinflussung der Schweißproduktion, insbesondere zu einer Verminderung der Schweißproduktion, führt. Somit wird selbst bei Abwesenheit schweißhemmender Halogenide und/oder Hydroxyhalogenide von Aluminium und/oder Zirconium eine effektive Verminderung des Achselschweißes gewährleistet.

Unter dem Begriff "schweißhemmend" wird erfindungsgemäß die Verminderung bzw. Reduzierung der Transpiration der Schweißdrüsen des Körpers verstanden.

Darüber hinaus werden unter dem Begriff "Halogenide und/oder Hydroxyhalogenide von Aluminium und/oder Zirconium" im Rahmen der vorliegenden Erfindung insbesondere Chloride, Bromide und lodide des Aluminiums und des Zirconiums sowie Verbindungen der Formeln Al(OH)_{y}X und Zr(OH)_{z}X verstanden, wobei X in den vorgenannten Formeln für ein Halogenidion steht.

Weiterhin wird unter dem Begriff "kosmetisches Öl" im Sinne der vorliegenden Erfindung ein für die kosmetische Verwendung geeignetes Öl verstanden, welches mit Wasser nicht in allen Mengen mischbar ist. Bei dem erfindungsgemäß verwendeten kosmetischen Öl handelt es sich weder um Riechstoffe, noch um ätherische Öle.

Zudem werden unter dem Begriff "Riechstoffe" im Sinne der vorliegenden Erfindung Substanzen mit einer Molmasse von 74 bis 300 g/mol verstanden, welche mindestens eine osmophore Gruppe im Molekül enthalten und einen Geruch und/oder Geschmack aufweisen, d. h. sie sind in der Lage, die Rezeptoren der Haarzellen des olfaktorischen Systems zu erregen. Osmophore Gruppen sind kovalent an das Molekülgerüst gebundene Gruppen in Form von Hydroxygruppen, Formylgruppen, Oxogruppen, Alkoxycarbonylgruppen, Nitrilgruppen, Nitrogruppen, Azidgruppen etc. In diesem Zusammenhang fallen unter den Begriff "Riechstoffe" im Sinne der vorliegenden Erfindung auch bei 20 °C und 1.013 hPa flüssige Parfümöle, Parfüme, oder Parfümölbestandteile.

Darüber hinaus werden unter dem Begriff "Wachse" im Rahmen der vorliegenden Erfindung Substanzen verstanden, welche bei 20 °C knetbar oder fest bis brüchig hart sind, eine grobe bis feinkristalline Struktur aufweisen und farblich durchscheinend bis opak, aber nicht glasartig sind. Weiterhin schmelzen diese Substanzen über 25 °C ohne Zersetzung, sind wenig oberhalb des Schmelzpunktes leicht flüssig (wenig viskos), weisen eine stark temperaturabhängige Konsistenz und Löslichkeit auf und sind unter leichtem Druck polierbar.

Der Begriff "Protein" bezeichnet erfindungsgemäß chemische Verbindungen, welche durch Peptid-Bindungen Säureamid-artig verknüpfte Kondensationsprodukte von Aminosäuren bilden. Die Anzahl der Aminosäuren in den Proteinen beträgt bevorzugt mindestens 2 und maximal 1.000 Aminosäuren. Unter dem Begriff "Protein" sind erfindungsgemäß auch Hydrolysate eines Proteins zu verstehen, welche Proteinfraktionen mit verschiedenen Aminosäuresequenzen und Molekulargewichten enthalten. Darüber hinaus sind unter diesem Begriff im Rahmen der vorliegenden Erfindung auch Mischungen von in Hülsenfrüchten der Gattung *Pisum* vorkommenden Proteinen zu verstehen.

Weiterhin sind unter dem Begriff "Hülsenfrüchte" im Rahmen der vorliegenden Erfindung Pflanzen zu verstehen, welche aus nur einem Fruchtblatt eine Hülsenfrucht (auch Legumen genannt) ausbilden, die bei der Reife an Bauch- und Rückennaht aufplatzt. Unter diesem Begriff werden erfindungsgemäß jedoch auch die Samen, insbesondere die reifen Samen, der zuvor angeführten Pflanzen verstanden.

Zudem sind unter dem Begriff "Kreuzblütengewächse" im Rahmen der vorliegenden Erfindung Pflanzen aus der Ordnung der Kreuzblütenartigen (Brassicales) zu verstehen, welche zwittrige vierzählige Blüten aufweisen. Zu den bekanntesten Kulturpflanzen in der Familie der Kreuzblütengewächse zählen beispielsweise der Raps, Kohlsorten, wie Blumenkohl, Rotkohl, Wirsing, Weißkohl, Spitzkohl, Rosenkohl, Kohlrabi, Grünkohl oder Broccoli, der Senf oder die Rübe, wie beispielsweise die Steckrübe. Besonders bevorzugt im Rahmen der vorliegenden Erfindung werden Proteine, welche aus der Kulturpflanze Raps isoliert werden können, eingesetzt.

Darüber hinaus wird unter dem Begriff "Veränderung der Lichtabsorption des mindestens einen Proteins" sowohl die positive und negative Veränderung der Lichtdurchlässigkeit der Probenmischung, insbesondere der Proteinlösung, als auch die Absorption von Licht durch das mindestens eine Protein bzw. die Probenmischung verstanden.

Weiterhin wird unter dem Begriff "pH-Wert Änderung" die kontinuierliche Veränderung des pH-Wertes verstanden. Die kontinuierliche Veränderung des pH-Wertes kann beispielsweise durch Titration, bzw. gleichmäßige Zugabe, einer Base oder Säure erreicht werden.

Der Begriff "Probenmischung" bezeichnet erfindungsgemäß eine Mischung aus dem mindestens einen speziellen Protein mit einem Lösungsmittel, insbesondere Wasser, Puffer oder salzhaltigen wässrigen Lösungen.

Zudem sind unter dem Begriff der "Fettsäuren", wie er im Rahmen der vorliegenden Erfindung verwendet wird, aliphatische Carbonsäuren zu verstehen, welche unverzweigte oder verzweigte Kohlenstoffreste mit 4 bis 40 Kohlenstoffatomen aufweisen. Die im Rahmen der vorliegenden Erfindung eingesetzten Fettsäuren können sowohl natürlich vorkommende als auch synthetisch hergestellte Fettsäuren sein. Weiterhin können die Fettsäuren einfach oder mehrfach ungesättigt sein.

Schließlich sind unter dem Begriff der "Fettalkohole" im Rahmen der vorliegenden Erfindung aliphatische, einwertige, primäre Alkohole zu verstehen, welche unverzweigte oder verzweigte Kohlenwasserstoffreste mit 4 bis 40 Kohlenstoffatomen aufweisen. Die im Rahmen der Erfindung eingesetzten Fettalkohole können auch ein- oder mehrfach ungesättigt sein.

Die Angabe Gew.-% bezieht sich vorliegend, sofern nicht anderes angegeben ist, auf das Gesamtgewicht der erfindungsgemäß verwendeten schweißhemmenden Kombination.

Als ersten Bestandteil a) enthält die erfindungsgemäß verwendete Kombination mindestens einen Stoff, welcher aus der Gruppe von kosmetischen Ölen, welche bei 20 °C und 1.013 hPa flüssig sind, Riechstoffen und Wachsen ausgewählt ist.

Im Rahmen der vorliegenden Erfindung ist das bei 20°C und 1.013 hPa flüssige kosmetische Öl ausgewählt aus der Gruppe von (i) flüchtigen cyclischen Siliconölen, insbesondere cylischen und linearen Siliconölen; (ii) flüchtigen Nichtsiliconölen, insbesondere flüssigen Paraffinölen und Isoparaffinölen; (iii) nichtflüchtigen Siliconölen; (iv) nichtflüchtigen Nichtsiliconölen; sowie (v) deren Mischungen.

Der Begriff "flüchtiges Öl" bezeichnet erfindungsgemäß Öle, welche bei 20 °C und einem Umgebungsdruck von 1.013 hPa einen Dampfdruck von 2,66 Pa bis 40.000 Pa (0,02 bis 300 mm Hg), vorzugsweise von 10 bis 12.000 Pa (0,1 bis 90 mm Hg), weiter bevorzugt von 13 bis 3.000 Pa (0,1 bis 23 mm Hg), insbesondere von 15 bis 500 Pa (0,1 bis 4 mm Hg), aufweisen.

Darüber hinaus werden unter dem Begriff "nichtflüchtige Öle" im Sinne der vorliegenden Erfindung Öle verstanden, welche bei 20 °C und einem Umgebungsdruck von 1.013 hPa einen Dampfdruck von weniger als 2,66 Pa (0,02 mm Hg) aufweisen.

Es kann erfindungsgemäß bevorzugt sein, Mischungen von flüchtigen Siliconölen und flüchtigen Nichtsiliconölen in den erfindungsgemäßen schweißhemmenden kosmetischen Mitteln einzusetzen, da hierdurch ein trockeneres Hautgefühl erreicht wird. Weiterhin kann es im Rahmen der vorliegenden Erfindung bevorzugt sein, wenn die schweißhemmenden kosmetischen Mittel ein nichtflüchtiges Siliconöl und/oder ein nichtflüchtiges Nichtsiliconöl enthalten, um unlösliche Bestandteile, wie Talkum oder auf der Haut angetrocknete Inhaltsstoffe, zu maskieren.

Erfindungsgemäß besonders bevorzugt ist der Einsatz von Mischungen von nichtflüchtigen und flüchtigen kosmetischen Ölen, da auf diese Weise Parameter wie Hautgefühl, Sichtbarkeit des Rückstands und Stabilität der erfindungsgemäß verwendeten schweißhemmenden Kombination eingestellt und das Mittel somit besser an die Bedürfnisse der Verbraucher angepasst werden kann.

Die im Rahmen der vorliegenden Erfindung einsetzbaren flüchtigen und nichtflüchtigen Siliconöle sowie flüchtigen und nichtflüchtigen Nichtsiliconöle sind beispielsweise in den Offenlegungsschriften DE 10 2010 063 250 A1 und DE 10 2012 222 692 A1 offenbart.

Gemäß einer bevorzugten Ausführungsform der vorliegenden Erfindung ist das bei 20 °C und 1.013 hPa flüssige kosmetische Öl in einer Gesamtmenge von 0,02 bis 98 Gew.-%, vorzugsweise von 2 bis 85 Gew.-%, bevorzugt von 4 bis 75 Gew.-%, weiter bevorzugt von 6 bis 70 Gew.-%, noch weiter bevorzugt von 8 bis 60 Gew.-%, insbesondere von 8 bis 20 Gew.-%, bezogen auf das Gesamtgewicht erfindungsgemäß verwendeten schweißhemmenden Kombination, enthalten.

Als Bestandteil a) erfindungsgemäß verwendeten schweißhemmenden Kombination kann ebenfalls mindestens ein Riechstoff enthalten sein. Bevorzugt werden jedoch Mischungen verschiedener Riechstoffe verwendet, welche gemeinsam eine ansprechende Duftnote erzeugen. Im Rahmen der vorliegenden Erfindung einsetzbare Riechstoffe sind beispielsweise in der Offenlegungsschrift DE 10 2010 063 250 A1 offenbart.

Besonders ansprechend riechende erfindungsgemäß verwendete schweißhemmende Kombinationen werden erhalten, wenn der mindestens eine Riechstoff in einer Gesamtmenge von 0,00001 bis 15 Gew.-%, vorzugsweise von 0,001 bis 9 Gew.-%, bevorzugt von 0,01 bis 8 Gew.-%, weiter bevorzugt von 0,1 bis 7 Gew.-%, noch weiter bevorzugt von 0,2 bis 6 Gew.-%, insbesondere von 0,2 bis 2 Gew.-%, bezogen auf das Gesamtgewicht der erfindungsgemäß verwendeten schweißhemmenden Kombination, enthalten ist.

Weiterhin können die erfindungsgemäß verwendeten schweißhemmenden Kombinationen als Bestandteil a) ein Wachs enthalten. Bevorzugt ist dieses Wachs ausgewählt aus der Gruppe von (i) Fettsäureglycerinmono-, -di- und -triestern; (ii) Butyrospermum Parkii (Shea Butter); (iii) Estern von gesättigten, einwertigen C₈₋₁₈-Alkoholen mit gesättigten C₁₂₋₁₈-Monocarbonsäuren; (iv) linearen, primären C₁₂₋₂₄-Alkanolen; (v) Estern aus einem gesättigten, einwertigen C₁₆₋₆₀-Alkanol und einer gesättigten C₈₋₃₆-Monocarbonsäure; (vi) Glycerintriestern von gesättigten linearen C₁₂₋₃₀-Carbonsäuren, die hydroxyliert sein können; (vii) natürlichen pflanzlichen Wachsen; (viii) tierischen Wachsen; (ix) synthetischen Wachsen; sowie (x) deren Mischungen. Im Rahmen der vorliegenden Erfindung bevorzugt einsetzbare Wachse sind in der Offenlegungsschrift DE 10 2012 222 692 A1 offenbart.

Im Rahmen der vorliegenden Erfindung ist es bevorzugt, wenn das Wachs in einer Gesamtmenge von 0,01 bis 50 Gew.-%, vorzugsweise von 3 bis 40 Gew.-%, bevorzugt von 5 bis 30 Gew-%, insbesondere von 6 bis 25 Gew.-%, bezogen auf das Gesamtgewicht der erfindungsgemäß verwendeten schweißhemmenden Kombination, enthalten ist.

Gemäß einer Ausführungsform der vorliegenden Erfindung kann es vorgesehen sein, dass die erfindungsgemäß verwendeten schweißhemmenden Kombinationen als Bestandteil b) ein Treibmittel in einer Gesamtmenge von 0 bis 99 Gew.-%, bezogen auf das Gesamtgewicht der erfindungsgemäß verwendeten schweißhemmenden Kombination, enthalten. Wenn die erfindungsgemäß verwendeten schweißhemmenden Kombinationen ein Treibmittel enthalten, ist dieses bevorzugt in einer Gesamtmenge von 1 bis 98 Gew.-%, vorzugsweise von 20 bis 90 Gew.-%, bevorzugt von 30 bis 85 Gew-%, insbesondere von 40 bis 75 Gew.-%, bezogen auf das Gesamtgewicht der erfindungsgemäß verwendeten schweißhemmenden Kombination, enthalten. In diesem Fall sind die erfindungsgemäß verwendeten schweißhemmenden Kombinationen als treibgasgetriebene Aerosole konfektioniert. Bevorzugte Treibmittel (Treibgase) sind Propan, Propen, n-Butan, iso-Butan, iso-Buten, n-Pentan, Penten, iso-Pentan, iso-Penten, Methan, Ethan, Dimethylether, Stickstoff, Luft, Sauerstoff, Lachgas, 1,1,1,3-Tetrafluorethan, Heptafluoro-n-propan, Perfluorethan, Monochlordifluormethan, 1,1-Difluorethan, Tetrafluoropropene und zwar sowohl einzeln als auch in deren Mischungen. Auch hydrophile Treibgase, wie z. B. Kohlendioxid, können vorteilhaft im Sinne der vorliegenden Erfindung eingesetzt werden, wenn der Anteil an hydrophilen Gasen gering gewählt wird und lipophiles Treibgas (z. B. Propan/Butan) im Überschuss vorliegt. Besonders bevorzugt sind Propan, n-Butan, iso-Butan sowie Mischungen dieser Treibgase. Es hat sich gezeigt, dass der Einsatz von n-Butan als einzigem Treibgas erfindungsgemäß besonders bevorzugt sein kann.

Als dritten Bestandteil c) enthält die erfindungsgemäß verwendete schweißhemmende Kombination mindestens ein spezielles Protein aus Hülsenfrüchten der Gattung *Pisum.* Die Gattung mit der lateinischen Bezeichnung *Pisum* bezeichnet Hülsenfrüchte in Form von Erbsen und umfasst beispielsweise die Schalerbse (auch Pahl-, Pal- oder Kneifelerbse genannt), die Markerbse, die Zuckererbse (auch Kaiserschote, Kiefelerbse, Kefe oder Zuckerschote genannt) sowie die Riesenerbse.

Eine besonders effektive Verminderung des Achselschweißes durch das mindestens eine spezielle Protein wird im Rahmen der vorliegenden Erfindung erreicht, wenn das mindestens eine Protein in einer Gesamtmenge von 0,5 bis 60 Gew.-%, vorzugsweise von 1,0 bis 50 Gew.-%, bevorzugt von 1,5 bis 40 Gew-%, weiter bevorzugt von 2,0 bis 30 Gew.-%, insbesondere von 2,0 bis 20 Gew.-%, bezogen auf das Gesamtgewicht der erfindungsgemäß verwendeten schweißhemmenden Kombination, enthalten ist. Ohne sich auf diese Theorie beschränken zu wollen resultiert der Einsatz der vorstehend genannten Mengen des mindestens einen speziellen Proteins in einer signifikanten Beeinflussung der Schweißdrüse(n) durch Geldbildung des Proteins in den Ausführungsgängen der Schweißdrüsen oder durch Beeinflussung des Ladungsgleichgewichts innerhalb der Schweißdrüse(n). Auf diese Weise wird eine hervorragende schweißhemmende Wirkung gewährleistet. Weiterhin führt der Einsatz der zuvor genannten Mengen des mindestens einen speziellen Proteins nicht zu instabilen Formulierungen, so dass die Stabilität der erfindungsgemäßen schweißhemmenden kosmetischen Mittel selbst über lange Lagerungszeiträume gewährleistet wird.

Besonders gute Ergebnisse im Hinblick auf die Verminderung und/oder Reduzierung der Achselnässe sowie auf die Hautverträglichkeit und die Lagerstabilität werden erhalten, wenn das mindestens eine Protein ein mittleres Molekulargewicht M_{w} von 150 bis 100.000 Da, vorzugsweise von 180 bis 50.000 Da, bevorzugt von 200 bis 10.000 Da, weiter bevorzugt von 250 bis 8.000 Da, insbesondere von 300 bis 5.000 Da, aufweist. Das mittlere Molekulargewicht M_{w} kann beispielsweise durch Gelpermeationschromatographie (GPC) bestimmt werden (Andrews P.; "Estimation of the Molecular Weights of Proteins by Sephadex Gel-Filtration"; Biochem. J., 1964, 91, Seiten 222 bis 233).

Gemäß einer bevorzugten Ausführungsform der vorliegenden Erfindung weist das mindestens eine Protein einen isoelektrischen Punkt auf, welcher im Bereich von pH 4,0 bis pH 10,0, vorzugsweise von pH 4,0 bis pH 9,5, insbesondere von pH 4,0 bis pH 8,0, liegt. Insbesondere Proteine, welche einen isoelektrischen Punkt im vorgenannten pH-Bereich aufweisen, haben sich im Rahmen der vorliegenden Erfindung als vorteilhaft im Hinblick auf die schweißhemmende Wirkung sowie die Stabilität der erfindungsgemäßen kosmetischen Mittel erwiesen.

Eine besonders hohe schweißhemmende Wirkung, Hautverträglichkeit sowie Lagerstabilität wird im Rahmen der vorliegenden Erfindung gewährleistet, wenn das mindestens eine Protein eine Veränderung der Lichtabsorption bei einer pH-Wert Änderung von mindestens 0,5 in einem pH-Bereich von pH 4,5 bis pH 7,5, insbesondere von pH 5,0 bis pH 7,0, bei einer Konzentration von 0,001 bis 10 Gew.-% Protein, bezogen auf das Gesamtgewicht der zur pH-Messung verwendeten Probenmischung, und einer Temperatur von 20 °C bewirkt. Ohne sich auf diese Theorie beschränken zu wollen resultiert der Einsatz des mindestens einen speziellen Proteins, welches in einem bestimmten pH-Bereich eine Veränderung der Lichtabsorption bewirkt, in einer signifikant erhöhten Beeinflussung der Schweißdrüse(n) durch pH-selektive Gelbildung in den Ausführungsgängen der Schweißdrüsen oder durch Störung des Ladungsgleichgewichts der Schweißdrüse(n), so dass eine hervorragende schweißhemmende Wirkung der erfindungsgemäßen kosmetischen Mittel gewährleistet wird, welche mit der schweißhemmenden Wirkung von aluminiumsalzhaltigen oder aluminium-zirkoniumsalzhaltigen kosmetischen Mitteln des Standes der Technik vergleichbar ist.

Im Rahmen der vorliegenden Erfindung ist es bevorzugt, wenn die pH-Wert Änderung durch Zugabe von Hydrogencarbonaten oder Carbonaten, insbesondere von Natriumhydrogencarbonaten, erreicht wird.

Gemäß einer bevorzugten Ausführungsform der vorliegenden Erfindung ist das mindestens eine Protein ausgewählt aus der Gruppe von (i) nicht modifizierten Proteinen; (ii) hydrolysierten Proteinen; (iii) chemisch modifizierten Proteinen, insbesondere hydrophob und/oder kationisch und/oder anionisch modifizierten Proteinen; (iv) physikalisch modifizierten Proteinen, insbesondere fraktionierten und/oder gereinigten und/oder bestrahlten Proteinen; (v) hydrolysierten nicht modifizierten Proteinen; (vi) hydrolysierten und chemisch modifizierten Proteinen, insbesondere hydrolysierten und hydrophob und/oder kationisch und/oder anionisch modifizierten Proteinen; (vii) hydrolysierten und physikalisch modifizierten Proteinen, insbesondere fraktionierten und/oder gereinigten und/oder bestrahlten Proteinen; sowie (viii) deren Mischungen.

Unter dem Begriff "nicht modifizierte Proteine" sind erfindungsgemäß Proteine zu verstehen, welche weder mittels chemischer Verfahren, wie beispielsweise der Hydrolyse oder der chemischen Modifikation, noch mittels physikalischer Methoden, wie beispielsweise der Aufreinigung, der Trennung sowie der Bestrahlung, behandelt wurden.

Weiterhin sind unter dem Begriff "hydrolysierte Proteine" bzw. "Proteinhydrolysate" erfindungsgemäß Proteine zu verstehen, welche durch chemische, insbesondere alkalische oder saure Hydrolyse, durch enzymatische Hydrolyse und/oder durch eine Kombination aus beiden Hydrolysearten hergestellt werden. Für den enzymatischen Abbau sind alle hydrolytisch wirkenden Enzyme geeignet, wie beispielsweise alkalische Proteasen. Übersichten zu Herstellung von Proteinhydrolysaten sind beispielsweise von G. Schuster und A. Domsch in Seifen Öle Fette Wachse 108, (1982) 177 bzw. Cosm.Toil. 99, (1984) 63, von H. W. Steisslinger in Parf.Kosm. 72, (1991) 556 und F. Aurich et al. in Tens.Surf.Det. 29, (1992) 389 erschienen. Mischungen von einzelnen Aminosäuren, welche lediglich durch Vermischen der Reinsubstanzen der Aminosäuren erhalten werden, sowie Totalhydrolysate, welche lediglich aus einzelnen Aminosäuren bestehen, fallen im Rahmen der vorliegenden Erfindung nicht unter den Begriff "hydrolysierte Proteine" bzw. "Proteinhydrolysate".

Darüber hinaus sind unter dem Begriff "chemisch modifizierte Proteine" im Rahmen der vorliegenden Erfindung Proteine zu verstehen, welche durch chemische Umsetzung der reaktiven Gruppen der Proteine, insbesondere der Hydroxy-, Amin-, Imidazol-, Guanidino- und/oder Thiolgruppen der Seitenketten der Aminosäuren des Proteins, mit hydrophoben und/oder kationischen und/oder anionischen Verbindungen erhalten werden.

Zudem sind unter dem Begriff "physikalisch modifizierte Proteine" im Sinne der vorliegenden Erfindung Proteine zu verstehen, welche durch physikalische Einwirkung, insbesondere durch Wärme und/oder Licht und/oder Fraktionierung, modifiziert wurden.

Im Rahmen dieser Ausführungsform ist es besonders bevorzugt, wenn das mindestens eine Protein ausgewählt ist aus der Gruppe von chemisch modifizierten, insbesondere hydrophob modifizierten, Proteinen. In diesem Zusammenhang weist das hydrophob modifizierte Protein ein oder mehrere C₄₋₃₀-Kohlenstoffketten auf, wobei die C₄₋₃₀-Kohlenwasserstoffketten linear, cyclisch, verzweigt, unverzweigt, gesättigt, ungesättigt und aromatisch sein können und wobei die C₄₋₃₀-Kohlenwasserstoffketten über Ether- und/oder Ester- und/oder Amin- und/oder Amidbindungen an den Proteinrest gebunden sind.

Darüber hinaus ist es im Rahmen dieser Ausführungsform bevorzugt, wenn das mindestens eine Protein ausgewählt ist aus der Gruppe von chemisch modifizierten, insbesondere kationisch modifizierten, Proteinen. Bevorzugt enthält das kationisch modifizierte Protein daher einen oder mehrere Rest(e) der Formel R¹-N⁺(CH₃)₂-CH₂-CH(OH)-CH₂-X-R, in welcher R¹ für eine Alkylgruppe mit 1 bis 30 Kohlenstoffatomen, eine Alkenylgruppe mit 1 bis 30 Kohlenstoffatomen, eine Hydroxyalkylgruppe mit 1 bis 30 Kohlenstoffatomen, insbesondere für eine Methylgruppe, eine C₁₀₋₁₈-Alkyl oder eine C₁₀₋₁₈-Alkenylgruppe, X für O, N oder S und R für den Proteinrest stehen. Die Kationisierung der Proteine mit den vorstehend beschriebenen Resten kann durch Umsetzung der Proteine mit den entsprechenden Halogeniden der obigen Formel erreicht werden, wobei die vorstehend beschriebenen Reste über Ether- und/oder Ester- und/oder Amid- und/oder Aminbindungen an das Protein gebunden sein können. Unter dem Begriff "Proteinrest" ist im Rahmen der vorliegenden Erfindung das durch die Verknüpfung von Aminosäuren gebildete Rückgrat des entsprechenden Proteins, an welches die kationische Gruppe über die zuvor genannten Bindungen gebunden ist, zu verstehen.

Im Hinblick auf die schweißhemmende Wirkung, die Hautverträglichkeit sowie die Lagerstabilität der erfindungsgemäß verwendeten Kombination ist es besonders bevorzugt, wenn das mindestens eine Protein ein aus Hülsenfrüchten der Gattung *Pisum* isoliertes Protein ist, wobei das Protein hydrolysiert ist und gegebenenfalls kationisch modifiziert ist. Ohne sich auf diese Theorie beschränken zu wollen resultiert der Einsatz dieser speziellen Proteine in einer signifikant erhöhten Beeinflussung der Schweißdrüse(n) durch pH-selektive Gelbildung oder Störung des Ladungsgleichgewichts innerhalb der Ausführungsgänge der Schweißdrüsen. Auf diese Weise wird eine hervorragende schweißhemmende Wirkung der erfindungsgemäß verwendeten Kombination gewährleistet, welche mit der schweißhemmenden Wirkung von aluminiumsalzhaltigen oder aluminium-zirkoniumsalzhaltigen kosmetischen Mitteln des Standes der Technik vergleichbar ist. Darüber hinaus führt der Einsatz dieser speziellen Proteine nicht zu einer negativen Wechselwirkung mit weiteren Inhaltsstoffen in dem schweißhemmenden kosmetischen Mittel, so dass eine hohe Lagerstabilität der erfindungsgemäßen schweißhemmenden kosmetischen Mittel gewährleistet wird. Zudem weisen die erfindungsgemäßen kosmetischen Mittel eine hohe Hautverträglichkeit auf.

Gemäß einer weiteren besonders bevorzugten Ausführungsform der vorliegenden Erfindung bewirkt das mindestens eine Protein eine Veränderung der Lichtabsorption von 1,5 bis 90 %, vorzugsweise von 2 bis 80 %, bevorzugt von 2,5 bis 70 %, weiter bevorzugt von 3 bis 65 %, insbesondere von 3,5 bis 60 %. Insbesondere Proteine aus Hülsenfrüchten der Gattung *Pisum,* welche die zuvor genannte Veränderung der Lichtabsorption bewirken, führen im Rahmen der vorliegenden Erfindung zu einer hervorragenden schweißhemmenden Wirkung. Die Veränderung der Lichtabsorption kann dabei auf einer Veränderung der Lichtdurchlässigkeit der Probenmischung, insbesondere durch Trübung, als auch auf die Absorption von Licht durch die Probenmischung, insbesondere durch das Protein selbst, erfolgen.

Die dieser Erfindung zugrundeliegenden Veränderungen der Lichtabsorption bei einer pH-Wert Änderung von mindestens 0,5 können durch Messung der Lichttransmission eines Lichtstrahls durch die Probenmischung bestimmt werden. Die Messungen der Lichttransmission wird unter Verwendung einer Methrom Optrode 6.1115.000 bei einer Wellenlänge von 574 nm (grüngelb) in mV (Auflösung 0,1 mV) in einem offenen Probengefäß bei 23 °C und 1.013 mbar durchgeführt. Die pH-Wert Änderung in dem pH-Bereich von 4,0 bis 8,0 wird durch langsame und kontinuierliche Zugabe einer Carbonat- oder Hydrogencarbonatlösung, bevorzugt einer 1 Gew.-%-igen Natriumhydrogencarbonatlösung, zu der Probenmischung unter Messung des pH-Werts mit einer pH-Elektrode sowie unter Rühren bei einer Geschwindigkeit von 750 bis 850 rpm erreicht. Die Veränderung der Lichtabsorption, welche durch das mindestens eine Protein bewirkt wird, berechnet sich nach der Formel ΔL = [(|Lᵢ|/|L₀|]^{∗}100. In dieser Formel steht Lᵢ für die Lichttransmission nach Veränderung des pH-Wertes um mindestens 0,5 in dem pH-Bereich von 4,0 bis 8,0, bevorzugt von pH 4,5 und 7,5, insbesondere von pH 5,0 und 7,0. L₀ steht in dieser Formel für die Differenz aus der Lichttransmission bei pH 4,0 und bei pH 8,0, bevorzugt bei pH 4,5 und bei pH 7,5, insbesondere bei pH 5,0 und bei pH 7,0, also beispielsweise Lichttransmission bei pH 8,0 minus Lichttransmission bei pH 4,0. Das mindestens eine spezielle Protein in den erfindungsgemäßen schweißhemmenden kosmetischen Mitteln bewirkt eine nach obiger Methode bestimmte Veränderung der Lichtabsorption von 1 bis 100 %. Die vorliegende Erfindung ist jedoch nicht auf schweißhemmende kosmetische Zusammensetzungen beschränkt, welche mindestens ein spezielles Protein enthalten, welches eine nach obiger Methode bestimmte Veränderung der Lichtabsorption von 1 bis 100 % bewirkt. Sie umfasst auch schweißhemmende kosmetische Zusammensetzungen, welche mindestens ein spezielles Protein enthalten, welches nach anderen Methoden eine Veränderung der Lichtabsorption von 1 bis 100 % bewirkt.

Im Rahmen der vorliegenden Erfindung ist es bevorzugt, wenn die Konzentration des mindestens einen Proteins in der zur Bestimmung der Veränderung der Lichtabsorption verwendeten Mischung von 0,005 bis 10 Gew.-%, vorzugsweise von 0,05 bis 5 Gew.-%, bevorzugt von 0,07 bis 3 Gew.-%, insbesondere von 0,09 bis 2 Gew.-%, bezogen auf das Gesamtgewicht der für die Bestimmung der Veränderung der Lichtabsorption verwendeten Probenmischung, beträgt.

Erfindungsgemäß bevorzugt bewirkt das mindestens eine Protein eine Veränderung der Lichtabsorption bei einer pH-Wert Änderung von mindestens 0,5 und höchstens 3,5, vorzugsweise von mindestens 0,5 und höchstens 2,5, insbesondere von mindestens 0,5 und höchstens 1,5. Die Änderung des pH-Wertes kann insbesondere durch die Zugabe von Säuren oder Basen, bevorzugt Basen in Form von Carbonaten oder Hydrogencarbonaten, in der entsprechenden Menge erreicht werden.

Gemäß einer weiteren bevorzugten Ausführungsform der vorliegenden Erfindung weist das schweißhemmende kosmetische Mittel einen pH-Wert von pH 2 bis pH 10 auf. Innerhalb dieses Bereichs ist eine stabile Formulierung der erfindungsgemäßen kosmetischen Mittel möglich, ohne dass unerwünschte Wechselwirkungen zwischen den Inhaltsstoffen der erfindungsgemäßen schweißhemmenden kosmetischen Mittel auftreten. Die Einstellung des gewünschten pH-Wertes kann erfindungsgemäß durch Verwendung von dem Fachmann bekannten und in schweißhemmenden kosmetischen Mitteln üblichen Säuren und Basen erfolgen.

Erfindungsgemäß ist es weiterhin bevorzugt, wenn das schweißhemmende kosmetische Mittel zusätzlich mindestens ein Konservierungsmittel enthält. Erfindungsgemäß bevorzugte Konservierungsmittel sind Formaldehydabspalter Iodopropinylbutylcarbamate, Parabene, Phenoxyethanol, Ethanol, Benzoesäure und deren Salze, Dibromdicyanobutan, 2-Brom-2-nitro-propan-1,3-diol, Imidazolidinylharnstoff, 5-Chlor-2-methyl-4-isothiazolin-3-on, 2-Chloracetamid, Benzalkoniumchlorid, Benzylalkohol, Salicylsäure und Salicylate. Weitere im Rahmen der vorliegenden Erfindung einsetzbare Konservierungsmittel sind die in Anlage 6 der Kosmetikverordnung aufgeführten Substanzen sowie kosmetische Rohstoffe mit konservierenden Eigenschaften oder Rohstoffe, welche die konservierende Wirkung der vorgenannten Konservierungsmittel unterstützen bzw. verstärken. Die Konservierungsmittel sind vorzugsweise in einer Gesamtmenge von 0,01 bis 10 Gew.-%, vorzugsweise von 0,1 bis 7 Gew.-%, bevorzugt von 0,2 bis 5 Gew.-%, insbesondere von 0,3 bis 2,0 Gew.-%, bezogen auf das Gesamtgewicht des schweißhemmenden kosmetischen Mittels, enthalten.

Im Rahmen der vorliegenden Erfindung ist es bevorzugt, wenn das schweißhemmende kosmetische Mittel als Wasser-in-ÖI-Emulsion vorliegt. Hierbei kann es sich insbesondere um eine versprühbare Wasser-in-ÖI-Emulsion handeln, welche mittels eines Treibmittels versprüht werden kann. In diesem Zusammenhang ist es bevorzugt, wenn das in Form einer Wasser-in-ÖI-Emulsion vorliegende erfindungsgemäße schweißhemmende kosmetische Mittel das mindestens eine Protein in einer Gesamtmenge von 0,1 bis 70 Gew.-%, vorzugsweise von 0,5 bis 60 Gew.-%, bevorzugt von 1,0 bis 50 Gew.-%, weiter bevorzugt von 1,5 bis 40 Gew-%, noch weiter bevorzugt von 2,0 bis 30 Gew.-%, insbesondere von 2,0 bis 20 Gew.-%, bezogen auf das Gesamtgewicht des schweißhemmenden kosmetischen Mittels, enthält.

Es kann jedoch erfindungsgemäß gleichermaßen bevorzugt sein, wenn das schweißhemmende kosmetische Mittel als Öl-in-Wasser-Emulsion vorliegt. In diesem Fall wird das erfindungsgemäße kosmetische Mittel bevorzugt als treibmittelfreies Pumpspray oder Quetschspray versprüht oder als Roll-on appliziert. In diesem Zusammenhang ist es bevorzugt, wenn das in Form einer Öl-in-Wasser-Emulsion vorliegende schweißhemmende kosmetische Mittel das mindestens eine Protein in einer Gesamtmenge von 0,1 bis 70 Gew.-%, vorzugsweise von 0,5 bis 60 Gew.-%, bevorzugt von 1,0 bis 50 Gew.-%, weiter bevorzugt von 1,5 bis 40 Gew-%, noch weiter bevorzugt von 2,0 bis 30 Gew.-%, insbesondere von 2,0 bis 20 Gew.-%, bezogen auf das Gesamtgewicht des schweißhemmenden kosmetischen Mittels, enthält.

Gemäß einer weiteren bevorzugten Ausführungsform der vorliegenden Erfindung können die erfindungsgemäßen kosmetischen Mittel nur einen geringen Gehalt an freiem Wasser bzw. kein freies Wasser enthalten. Unter freiem Wasser im Sinne der vorliegenden Erfindung wird Wasser verstanden, welches von Kristallwasser, Hydratationswasser oder ähnlich molekular gebundenem Wasser der eingesetzten Bestandteile verschieden ist. Das schweißhemmende kosmetische Mittel enthält bevorzugt freies Wasser in einer Gesamtmenge von weniger als 10 Gew.-%, vorzugweise von weniger als 8 Gew.-%, bevorzugt von weniger als 5 Gew.-%, weiter bevorzugt von weniger als 3 Gew.-%, noch mehr bevorzugt von weniger als 1 Gew.-%, insbesondere von 0 Gew.-%, bezogen auf das Gesamtgewicht des schweißhemmenden kosmetischen Mittels.

Im Rahmen einer weiteren Ausführungsform ist es jedoch erfindungsgemäß ebenfalls bevorzugt, wenn das schweißhemmende kosmetische Mittel als wässrige, wässrig-alkoholische oder wässrigglykolische Lösung vorliegt. Da die erfindungsgemäßen kosmetischen Mittel keine schweißhemmenden Halogenide und/oder Hydroxyhalogenide von Aluminium und/oder Zirconium enthalten, welche durch den Zusatz protischer Lösungsmittel eine verminderte schweißhemmende Wirkung aufweisen, können erfindungsgemäß protische Lösungsmittel, wie wässrige Lösungen, zur Formulierung der erfindungsgemäßen schweißhemmenden kosmetischen Mittel verwendet werden, ohne dass eine signifikante Verringerung der Antitranspirantwirkung auftritt. Daher gewährleistet der Zusatz des mindestens einen speziellen Proteins selbst bei Verwendung von protischen Lösungsmitteln eine effektive Beeinflussung der der Schweißdrüse(n) und somit eine hervorragende Antitranspirantwirkung.

Im Zusammenhang mit dieser Ausführungsform der vorliegenden Erfindung wurde überraschenderweise festgestellt, dass die Beeinflussung der Schweißdrüse(n) durch das mindestens eine spezielle Protein signifikant gesteigert werden kann, wenn die erfindungsgemäßen schweißhemmenden kosmetischen Mittel freies Wasser in einer Menge von 5 bis 99 Gew.-%, bezogen auf das Gesamtgewicht des schweißhemmenden kosmetischen Mittels, enthalten. In einer besonders bevorzugten Ausführungsform der vorliegenden Erfindung enthält das schweißhemmende kosmetische Mittel daher freies Wasser in einer Gesamtmenge von 5 bis 96 Gew.-%, vorzugsweise von 15 bis 80 Gew.-%, bevorzugt von 30 bis 70 Gew.-%, insbesondere von 40 bis 60 Gew.-%, bezogen auf das Gesamtgewicht des schweißhemmenden kosmetischen Mittels.

Weiterhin ist es im Zusammenhang mit dieser Ausführungsform bevorzugt, wenn das schweißhemmende kosmetische Mittel Ethanol in einer Gesamtmenge von 1 bis 99 Gew.-%, vorzugsweise von 5 bis 70 Gew.-%, bevorzugt von 7 bis 50 Gew.-%, insbesondere von 10 bis 30 Gew.-%, bezogen auf das Gesamtgewicht des schweißhemmenden kosmetischen Mittels, enthält. Wie zuvor ausgeführt, können durch den Einsatz des mindestens einen speziellen Proteins selbst hohe Mengen an protischen Lösungsmitteln, wie Ethanol, verwendet werden, ohne dass die Antitranspirantwirkung des erfindungsgemäßen schweißhemmenden kosmetischen Mittels negativ beeinflusst wird.

Die Applikation des erfindungsgemäßen schweißhemmenden kosmetischen Mittels kann mittels verschiedener Verfahren erfolgen. Gemäß einer bevorzugten Ausführungsform ist das schweißhemmende kosmetische Mittel als Spray-Applikation konfektioniert. Die Spray-Applikation erfolgt mit einer Sprühvorrichtung, welche in einem Behälter eine Füllung aus dem erfindungsgemäßen flüssigen, viskos-fließfähigen, suspensionsförmigen oder pulverförmigen schweißhemmenden kosmetischen Mittel enthält. Die Füllung kann unter dem Druck eines Treibmittels stehen (Druckgasdosen, Druckgaspackungen, Aerosolpackungen), oder es kann sich um einen mechanisch zu bedienenden Pumpzerstäuber ohne Treibgas (Pumpsprays/ Quetschflasche) handeln. Die Zerstäubung des schweißhemmenden kosmetischen Mittels kann hierbei physikalisch, mechanisch oder elektromechanisch, beispielsweise durch Piezzoeffekte oder elektrische Pumpen, erfolgen. Im Rahmen dieser Ausführungsform einsetzbare Behälter und Entnahmevorrichtungen sind beispielsweise in der Offenlegungsschrift DE 10 2012 222 692 A1 beschrieben.

Das schweißhemmende kosmetische Mittel kann weiterhin bevorzugt als Stift, Soft Solid, Creme, Gel, Roll-on, loses oder kompaktes Puder konfektioniert sein. Die Formulierung der erfindungsgemäßen schweißhemmenden kosmetischen Mittel in einer bestimmten Darreichungsform, wie beispielsweise einem Antitranspirant-Roll-on, einem Antitranspirantstift oder einem Antitranspirantgel, richtet sich bevorzugt nach den Anforderungen des Verwendungszwecks. Je nach Verwendungszweck können die erfindungsgemäßen schweißhemmenden kosmetischen Mittel daher in fester, halbfester, flüssiger, disperser, emulgierter, suspendierter, gelförmiger, mehrphasiger oder puderförmiger Form vorliegen. Unter den Begriff der Flüssigkeit fallen im Sinne der vorliegenden Erfindung auch jegliche Arten von Festkörperdispersionen in Flüssigkeiten. Weiterhin werden unter mehrphasigen erfindungsgemäßen schweißhemmenden kosmetischen Mitteln im Sinne der vorliegenden Erfindung Mittel verstanden, welche mindestens 2 verschiedene Phasen mit einer Phasentrennung aufweisen und bei welchen die Phasen horizontal, also übereinander, oder vertikal, also nebeneinander, angeordnet sein können. Die Applikation kann beispielsweise mit einem Rollkugelapplikator oder mittels eines festen Stifts erfolgen.

Es kann im Rahmen der vorliegenden Erfindung ebenfalls bevorzugt sein, wenn das schweißhemmende kosmetische Mittel auf und/oder in einem wegwerfbaren Substrat, ausgewählt aus der Gruppe von Tüchern, Pads und Bauschen, enthalten ist. Besonders bevorzugt sind Feuchttücher, d.h. für den Anwender vorgefertigte, bevorzugt einzeln abgepackte, Feuchttücher, wie sie z. B. aus dem Bereich der Glasreinigung oder aus dem Bereich der feuchten Toilettenpapiere wohlbekannt sind. Solche Feuchttücher, die vorteilhafter Weise auch Konservierungsstoffe enthalten können, sind mit einem erfindungsgemäßen schweißhemmenden kosmetischen Mittel imprägniert oder beaufschlagt und bevorzugt einzeln verpackt. Bevorzugte Substratmaterialien sind ausgewählt aus porösen flächigen Tüchern. Zu diesen Tüchern gehören Tücher aus gewebten und ungewebten (Vlies) synthetischen und natürlichen Fasern, Filz, Papier oder Schaumstoff, wie hydrophilem Polyurethanschaum. Erfindungsgemäß bevorzugte deodorierende oder schweißhemmende Substrate können durch Tränken oder Imprägnierung oder auch durch Aufschmelzen eines erfindungsgemäßen schweißhemmenden kosmetischen Mittels auf ein Substrat erhalten werden.

Erfindungsgemäß bevorzugt enthält das schweißhemmende kosmetische Mittel mindestens einen weiten Hilfsstoff, ausgewählt aus der Gruppe von (i) Emulgatoren und/oder Tensiden; (ii) Verdickungsmitteln; (iii) Chelatbildnern; (iv) Deodorant-Wirkstoffen; (v) ein- und/oder mehrwertigen Alkoholen und/oder Polyethylenglycolen; (vi) hautkühlenden Wirkstoffen; (vii) pH-Stellmitteln; (viii) hautpflegenden Wirkstoffen, wie Moisturizern, hautberuhigenden Stoffen, hautaufhellenden Stoffen, hautglättenden Stoffen; sowie (ix) deren Mischungen.

Erfindungsgemäß bevorzugt geeignete Emulgatoren und Tenside sind ausgewählt aus anionischen, kationischen, nichtionischen, amphoteren, insbesondere ampholytischen und zwitterionischen Emulgatoren und Tensiden. Tenside sind amphiphile (bifunktionelle) Verbindungen, die aus mindestens einem hydrophoben und mindestens einem hydrophilen Molekülteil bestehen. Der hydrophobe Rest ist bevorzugt eine Kohlenwasserstoffkette mit 8 bis 28 Kohlenstoffatomen, die gesättigt oder ungesättigt, linear oder verzweigt sein kann. Besonders bevorzugt ist diese C₈-C₂₈-Alkylkette linear. Im Rahmen der vorliegenden Erfindung bevorzugt einsetzbare Emulgatoren und Tenside sind beispielsweise in den Offenlegungsschriften DE 10 2012 222 692 A1, DE 10 2010 063 250 A1 sowie DE 10 2010 055 816 A1 offenbart.

Zur Verdickung der erfindungsgemäßen schweißhemmenden kosmetischen Mittel werden bevorzugt Substanzen eingesetzt, welche ausgewählt sind aus Celluloseethern, Xanthan-Gum, Sclerotium Gum, Succinoglucanen, Polygalactomannanen, Pectinen, Agar, Carragheen (Carrageenan), Traganth, Gummi arabicum, Karayagummi, Taragummi, Gellan, Gelatine, Propylenglycolalginat, Alginsäuren und deren Salze, Polyvinylpyrrolidonen, Polyvinylalkoholen, Polyacrylamiden, physikalisch (z. B. durch Vorverkleisterung) und/oder chemisch modifizierten Stärken, Acrylsäure-Acrylat-Copolymeren, Acrylsäure-Acrylamid-Copolymeren, Acrylsäure-Vinylpyrrolidon-Copolymeren, Acrylsäure-Vinylformamid-Copolymeren und Polyacrylaten. Besonders bevorzugte Verdickungsmittel sind weiterhin ausgewählt aus Carbomeren. Carbomere sind verdickende vernetzte Polymere aus Acrylsäure, Methacrylsäure sowie deren Salzen. Die Vernetzung kann mittels polyfunktioneller Verbindungen wie Polyalkylenether von Polysacchariden oder Polyalkoholen, beispielsweise Sucroseallylether, Pentaerythritolallylether, Propylenallylether, erfolgen. Bevorzugt im Rahmen der vorliegenden Erfindung sind Homopolymere von Acrylsäure oder deren Salzen, welche mit einem Pentaerythritolallylether, einem Sucroseallylether oder einem Propylenallylether vernetzt sind. Ein im Rahmen der vorliegenden Erfindung einsetzbares Verdickungsmittel ist ein Copolymer aus C₁₀₋₃₀-Alkylacrylat, Acrylsäure, Methacrylsäure sowie deren Estern, welches mit einem Sucroseallylether oder einem Pentaerythritolallylether vernetzt ist. Verdickungsmittel auf Basis von Carbomeren sind die unter dem Handelsnamen Carbopol® (BF Goodrich, Ohio, USA) erhältlichen Produkte wie beispielsweise Carbopol 934, Carbopol 940, Carbopol 941, Carbopol 971, Carbopol 974, Carbopol EZ2, Carbopol ETD 2001, Carbopol ETD 2020, Carbopol ETD 2050, Carbopol ultrez 10, Carbopol ultrez 20, oder Carbopol ultrez 21.

Weiterhin können zur Verdickung der erfindungsgemäßen schweißhemmenden kosmetischen Mittel lipophile Verdickungsmittel eingesetzt werden. Erfindungsgemäß bevorzugte lipophile Verdickungsmittel sind ausgewählt aus hydrophobierten Tonmineralien, Bentoniten, pyrogenen Kieselsäuren sowie deren Derivaten.

Um die Beeinflussung der Schweißdrüse(n) durch das mindestens eine spezielle Protein weiter zu unterstützen, kann es von Vorteil sein, den erfindungsgemäßen schweißhemmenden kosmetischen Mitteln mindestens einen Chelatbildner, in einer Gesamtmenge von 0,01 bis 3,0 Gew.-%, vorzugsweise von 0,02 bis 1,0 Gew.-%, insbesondere von 0,05 bis 0,1 Gew.-%, bezogen auf das Gesamtgewicht des erfindungsgemäßen schweißhemmenden Mittels, zuzusetzen. Im Rahmen der vorliegenden Erfindung sind bevorzugte Chelatbildner ausgewählt aus der Gruppe von β-Alanindiessigsäure, Cyclodextrin, Diethylentriaminpentamethylenphosphonsäure, Natrium-, Kalium-, Calciumdinatrium-, Ammonium- und Triethanolaminsalzen der Ethylendiamintetraessigsäure (EDTA), Etidronsäure, Hydroxyethylethylendiamintetraessigsäure (HEDTA) und ihren Natriumsalzen, Natriumsalzen der Nitrilotriessigsäure (NTA), Diethylentriaminpentaessigsäure, Phytinsäure, Hydroxypropylcyclodextrin, Methylcyclodextrin, Aminotrimethylenphosphonat-Pentanatrium, Ethylendiamintetramethylenphosphonat-Pentanatrium, Diethylentriamin-pentaacetat-Pentanatrium, Pentanatriumtriphosphat, Kalium-EDTMP, Natrium-EDTMP, Natriumdihydroxyethylglycinat, Natriumphytat, Natriumpolydimethylglycinophenolsulfonat, Tetrahydroxyethylethylendiamin, Tetrahydroxypropylethylendiamin, Tetrakaliumetidronat, Tetranatriumetidronat, Tetranatriumiminodisuccinat, Trinatriumethylendiamindisuccinat, Tetranatrium-N,N-bis(Carboxymethyl)glutamat, Tetranatrium-DL-Alanin-N,N-diacetat und Desferrioxamin.

Die desodorierende Wirkung der erfindungsgemäßen schweißhemmenden kosmetischen Mittel kann weitergehend gesteigert werden, wenn mindestens ein Deodorant-Wirkstoff mit antibakterieller und/oder bakteriostatischer und/oder enzyminhibierender und/oder geruchsneutralisierender und/oder geruchsabsorbierender Wirkung in einer Gesamtmenge von 0,0001 bis 40 Gew.-%, vorzugsweise von 0,2 bis 20 Gew.-%, bevorzugt von 1 bis 15 Gew.-%, insbesondere von 1,5 bis 5 Gew.-%, bezogen auf das Gesamtgewicht des erfindungsgemäßen schweißhemmenden kosmetischen Mittels, enthalten ist. Sofern Ethanol in den erfindungsgemäßen Mitteln eingesetzt wird, gilt dieses im Rahmen der vorliegenden Erfindung nicht als Deodorant-Wirkstoff, sondern als Bestandteil des Trägers. Erfindungsgemäß bevorzugte Deodorant-Wirkstoffe sind beispielsweise in der Offenlegungsschrift DE 10 2010 063 250 A1 offenbart.

Bevorzugte erfindungsgemäße schweißhemmenden kosmetische Mittel enthalten weiterhin mindestens ein wasserlösliches mehrwertiges 6₂₋₉Alkanol mit 2 bis 6 Hydroxylgruppen und/oder mindestens ein wasserlösliches Polyethylenglycol mit 3 bis 50 Ethylenoxid-Einheiten sowie Mischungen hiervon. Hierunter fallen nicht die vorstehend erwähnten Deodorant-Wirkstoffe in Form von 1,2-Alkandiolen. Bevorzugte Alkanole und wasserlösliche Polyethylenglycole sind beispielsweise in der Offenlegungsschrift DE 10 2010 063 250 A1 beschrieben.

Gemäß einer weiteren Ausführungsform der vorliegenden Erfindung enthalten die schweißhemmenden kosmetischen Mittel weiterhin mindestens einen hautkühlenden Wirkstoff. Erfindungsgemäß geeignete hautkühlende Wirkstoffe sind beispielsweise Menthol, Isopulegol sowie Mentholderivate, z. B. Menthyllactat, Menthylglycolat, Menthyl Ethyl Oxamate, Menthylpyrrolidoncarbonsäure, Menthylmethylether, Menthoxypropandiol, Menthonglycerinacetal (9-Methyl-6-(1-methylethyl)-1,4-dioxaspiro (4.5)decan-2-methanol), Monomenthylsuccinat, 2-Hydroxymethyl-3,5,5-trimethylcyclohexanol und 5-Methyl-2-(1-methylethyl)cyclohexyl-N-ethyloxamat. Als hautkühlende Wirkstoffe bevorzugt sind Menthol, Isopulegol, Menthyllactat, Menthoxypropandiol, Menthylpyrrolidoncarbonsäure und 5-Methyl-2-(1-methylethyl)cyclohexyl-N-ethyloxamat sowie Mischungen dieser Substanzen, insbesondere Mischungen von Menthol und Menthyllactat, Menthol, Mentholglycolat und Menthyllactat, Menthol und Menthoxypropandiol oder Menthol und Isopulegol.

Als pH-Stellmittel kommen erfindungsgemäß bevorzugt Säuren und/oder Alkalisierungsmittel und/oder Puffer zum Einsatz. Als Säuren werden erfindungsgemäß bevorzugt anorganische Säuren (wie beispielsweise Salzsäure, Schwefelsäure oder Phosphorsäure) oder organische Säuren (wie beispielsweise Zitronensäure, Weinsäure oder Apfelsäure) eingesetzt. Die erfindungsgemäß verwendbaren Alkalisierungsmittel werden bevorzugt ausgewählt aus der Gruppe, welche gebildet wird, aus Ammoniak, basischen Aminosäuren, Alkalihydroxiden, Carbonaten und Hydrogencarbonaten, Alkanolaminen, beispielsweise Amino-2-methyl-1-propanol, Monoethanolamin, Triethanolamin, Diethanolamin und Triisopropanolamin, Alkalimetallmetasilikaten, Harnstoff, Morpholin, N-Methylglucamin, Imidazol, Alkaliphosphaten und Alkalihydrogenphosphaten. Als Alkalimetallionen dienen bevorzugt Lithium, Natrium, Kalium, insbesondere Natrium oder Kalium. Als Puffersysteme eignen sich im Rahmen der vorliegenden Erfindung insbesondere Kohlensäure-Bicarbonat-Puffer, Kohlensäure-Silicat-Puffer, Essigsäure-Acetat-Puffer, Phosphatpuffer, Ammoniakpuffer, Citronensäure- oder Citratpuffer, Puffer auf Basis von Tris(hydroxymethyl)-aminomethan, Puffer auf Basis von 4-(2-Hydroxyethyl)-1-piperazinethanesulfonsäure, Puffer auf Basis von 4-(2-Hydroxyethyl)-piperazin-1-propansulfonsäure, Puffer auf Basis von 2-(N-Morpholino)ethansulfonsäure sowie Barbital-Acetat-Puffer. Die Wahl des entsprechenden Puffersystems richtet sich hierbei nach dem gewünschten pH-Wert der erfindungsgemäß verwendeten schweißhemmenden kosmetischen Mittel.

In einer bevorzugten Ausführungsform sind die erfindungsgemäß verwendeten schweißhemmenden kosmetischen Mittel dadurch gekennzeichnet, dass sie - bezogen auf das Gesamtgewicht des erfindungsgemäß verwendeten schweißhemmenden kosmetischen Mittels -
- mindestens ein Protein in einer Gesamtmenge von 0,5 bis 60 Gew.-%, vorzugsweise von 1,0 bis 50 Gew.-%, bevorzugt von 1,5 bis 40 Gew-%, weiter bevorzugt von 2,0 bis 30 Gew.-%, insbesondere von 2,0 bis 20 Gew.-%,
- 12 bis 98 Gew.-%, vorzugsweise 25 bis 55 Gew.-%, bevorzugt 30 bis 50 Gew.-%, insbesondere 35 bis 45 Gew.-% Wasser,
- mindestens einen Emulgator und/oder ein Tensid,
- mindestens ein pH-Stellmittel,
- mindestens ein Konservierungsmittel und
- mindestens einen Stoff, ausgewählt aus der Gruppe von kosmetischen Ölen, welche bei 20 °C und 1.013 hPa flüssig sind, Riechstoffen und Wachsen
enthalten, wobei das mindestens eine Protein in Hülsenfrüchten der Gattung *Pisum* vorkommt und wobei das mindestens eine Protein bei einer pH-Wert Änderung von mindestens 0,5 in einem pH-Bereich von pH 4,0 bis pH 8,0, einer Temperatur von 20 °C bis 40 °C und einer Konzentration des Proteins von 0,001 bis 10 Gew.-%, bezogen auf das Gesamtgewicht der für die Bestimmung der Veränderung der Lichtabsorption verwendeten Probenmischung, eine Veränderung der Lichtabsorption von 1 bis 100 %, bewirkt.

In einer weiteren bevorzugten Ausführungsform sind die erfindungsgemäß verwendeten schweißhemmenden kosmetischen Mittel dadurch gekennzeichnet, dass sie - bezogen auf das Gesamtgewicht des erfindungsgemäß verwendeten schweißhemmenden kosmetischen Mittels -
- mindestens ein Protein in einer Gesamtmenge von 0,5 bis 60 Gew.-%, vorzugsweise von 1,0 bis 50 Gew.-%, bevorzugt von 1,5 bis 40 Gew-%, weiter bevorzugt von 2,0 bis 30 Gew.-%, insbesondere von 2,0 bis 20 Gew.-%,
- 12 bis 98 Gew.-%, vorzugsweise 25 bis 55 Gew.-%, bevorzugt 30 bis 50 Gew.-%, insbesondere 35 bis 45 Gew.-% Wasser,
- mindestens einen Emulgator und/oder ein Tensid,
- mindestens ein pH-Stellmittel,
- mindestens ein Konservierungsmittel,
- 0,01 bis 2 Gew.-%, vorzugsweise 0,1 bis 1 Gew.-%, bevorzugt 0,2 bis 0,7 Gew.-%, insbesondere 0,3 bis 0,5 Gew.-% eines Verdickungsmittels, und
- mindestens einen Stoff, ausgewählt aus der Gruppe von kosmetischen Ölen, welche bei 20 °C und 1.013 hPa flüssig sind, Riechstoffen und Wachsen
enthalten, wobei das mindestens eine Protein in Hülsenfrüchten der Gattung *Pisum* vorkommt und wobei das mindestens eine Protein bei einer pH-Wert Änderung von mindestens 0,5 in einem pH-Bereich von pH 4,0 bis pH 8,0, einer Temperatur von 20 °C bis 40 °C und einer Konzentration des Proteins von 0,001 bis 10 Gew.-%, bezogen auf das Gesamtgewicht der für die Bestimmung der Veränderung der Lichtabsorption verwendeten Probenmischung, eine Veränderung der Lichtabsorption von 1 bis 100 %, bewirkt.

In einer bevorzugten Ausführungsform sind die erfindungsgemäß verwendeten schweißhemmenden kosmetischen Mittel dadurch gekennzeichnet, dass sie - bezogen auf das Gesamtgewicht des erfindungsgemäß verwendeten schweißhemmenden kosmetischen Mittels -
- mindestens ein Protein in einer Gesamtmenge von 0,5 bis 60 Gew.-%, vorzugsweise von 1,0 bis 50 Gew.-%, bevorzugt von 1,5 bis 40 Gew-%, weiter bevorzugt von 2,0 bis 30 Gew.-%, insbesondere von 2,0 bis 20 Gew.-%,
- 12 bis 98 Gew.-%, vorzugsweise 25 bis 55 Gew.-%, bevorzugt 30 bis 50 Gew.-%, insbesondere 35 bis 45 Gew.-% Wasser,
- mindestens ein Treibmittel in einer Gesamtmenge von 1 bis 98 Gew.-%, vorzugsweise von 20 bis 90 Gew.-%, bevorzugt von 30 bis 85 Gew-%, insbesondere von 40 bis 75 Gew.-%,
- mindestens einen Emulgator und/oder ein Tensid,
- mindestens ein pH-Stellmittel,
- mindestens ein Konservierungsmittel und
- mindestens einen Stoff, ausgewählt aus der Gruppe von kosmetischen Ölen, welche bei 20 °C und 1.013 hPa flüssig sind, Riechstoffen und Wachsen
enthalten, wobei das mindestens eine Protein in Hülsenfrüchten der Gattung *Pisum* vorkommt und wobei das mindestens eine Protein bei einer pH-Wert Änderung von mindestens 0,5 in einem pH-Bereich von pH 4,0 bis pH 8,0, einer Temperatur von 20 °C bis 40 °C und einer Konzentration des Proteins von 0,001 bis 10 Gew.-%, bezogen auf das Gesamtgewicht der für die Bestimmung der Veränderung der Lichtabsorption verwendeten Probenmischung, eine Veränderung der Lichtabsorption von 1 bis 100 %, bewirkt.

In einer weiteren bevorzugten Ausführungsform sind die erfindungsgemäß verwendeten schweißhemmenden kosmetischen Mittel dadurch gekennzeichnet, dass sie - bezogen auf das Gesamtgewicht des erfindungsgemäß verwendeten schweißhemmenden kosmetischen Mittels -
- mindestens ein Protein in einer Gesamtmenge von 0,5 bis 60 Gew.-%, vorzugsweise von 1,0 bis 50 Gew.-%, bevorzugt von 1,5 bis 40 Gew-%, weiter bevorzugt von 2,0 bis 30 Gew.-%, insbesondere von 2,0 bis 20 Gew.-%,
- 12 bis 98 Gew.-%, vorzugsweise 25 bis 55 Gew.-%, bevorzugt 30 bis 50 Gew.-%, insbesondere 35 bis 45 Gew.-% Wasser,
- mindestens ein Treibmittel in einer Gesamtmenge von 1 bis 98 Gew.-%, vorzugsweise von 20 bis 90 Gew.-%, bevorzugt von 30 bis 85 Gew-%, insbesondere von 40 bis 75 Gew.-%,
- mindestens einen Emulgator und/oder ein Tensid,
- mindestens ein pH-Stellmittel,
- mindestens ein Konservierungsmittel,
- 0,01 bis 2 Gew.-%, vorzugsweise 0,1 bis 1 Gew.-%, bevorzugt 0,2 bis 0,7 Gew.-%, insbesondere 0,3 bis 0,5 Gew.-% eines Verdickungsmittels, und
- mindestens einen Stoff, ausgewählt aus der Gruppe von kosmetischen Ölen, welche bei 20 °C und 1.013 hPa flüssig sind, Riechstoffen und Wachsen
enthalten, wobei das mindestens eine Protein in Hülsenfrüchten der Gattung *Pisum* vorkommt und wobei das mindestens eine Protein bei einer pH-Wert Änderung von mindestens 0,5 in einem pH-Bereich von pH 4,0 bis pH 8,0, einer Temperatur von 20 °C bis 40 °C und einer Konzentration des Proteins von 0,001 bis 10 Gew.-%, bezogen auf das Gesamtgewicht der für die Bestimmung der Veränderung der Lichtabsorption verwendeten Probenmischung, eine Veränderung der Lichtabsorption von 1 bis 100 %, bewirkt.

Im Rahmen der vorliegenden Erfindung kann es auch vorgesehen sein, das erfindungsgemäß verwendete kosmetische Mittel als Zwei-Komponenten-Mittel zu konfektionieren. Die einzelnen Komponenten werden hierzu vorzugsweise in getrennten Containern gelagert und in beliebiger Reihenfolge nacheinander oder gleichzeitig auf die Haut aufgetragen. Eine Auftrennung in Mehrkomponentensysteme ist insbesondere dort bevorzugt, wo Inkompatibilitäten der Inhaltsstoffe zu erwarten oder zu befürchten sind.

Ein weiterer Gegenstand der vorliegenden Erfindung ist daher eine Verpackungseinheit (Kit-of-Parts), umfassend - getrennt voneinander konfektioniert -
a) mindestens einen ersten Container (C1), enthaltend ein kosmetisches Mittel (M1) umfassend mindestens einen schweißhemmenden Wirkstoff, und
b) mindestens einen zweiten Container (C2), enthaltend ein kosmetisches Mittel (M2) umfassend mindestens ein Protein, wobei das mindestens eine Protein ein aus Hülsenfrüchten der Gattung *Pisum* isoliertes Protein ist, wobei das Protein hydrolysiert ist und gegebenenfalls kationisch modifiziert ist
und wobei das mindestens eine Protein bei einer pH-Wert Änderung von mindestens 0,5 in einem pH-Bereich von pH 4,0 bis pH 8,0, einer Temperatur von 20 °C bis 40 °C und einer Konzentration des Proteins von 0,001 bis 10 Gew.-%, bezogen auf das Gesamtgewicht der für die Bestimmung der Veränderung der Lichtabsorption verwendeten Probenmischung, eine Veränderung der Lichtabsorption von 1 bis 100 % - gemessen wie in der Beschreibung angegeben - bewirkt
und wobei das kosmetische Mittel (M2) keine Halogenide und/oder Hydroxyhalogenide von Aluminium und/oder Zirconium enthält.

Unter dem Begriff "schweißhemmender Wirkstoff" werden erfindungsgemäß Wirkstoffe verstanden, welche die Transpiration der Schweißdrüsen des Körpers vermindern bzw. reduzieren. Hierunter fallen jedoch nicht die in dem kosmetischen Mittel (M2) enthaltenen Proteine aus Hülsenfrüchten der Gattung *Pisum* und/oder *Phaseolus* und/oder *Vigna* und/oder *Macrotyloma* oder aus Kreuzblütengewächsen der Gattung *Brassica,* welche unter den oben beschriebenen Bedingungen eine Veränderung der Lichtabsorption bewirken.

Bezüglich des kosmetischen Mittels (M2) in dem Container (C2) gilt mutatis mutandis das zu den erfindungsgemäßen kosmetischen Mitteln Gesagte.

Ein weiterer Gegenstand der vorliegenden Erfindung ist die Verwendung mindestens eines Proteins zur zumindest teilweisen Beeinflussung der Schweißdrüse(n), wobei das mindestens eine Protein ein aus Hülsenfrüchten der Gattung *Pisum* isoliertes Protein ist, wobei das Protein hydrolysiert ist und gegebenenfalls kationisch modifiziert ist und wobei das mindestens eine Protein bei einer pH-Wert Änderung von mindestens 0,5 in einem pH-Bereich von pH 4,0 bis pH 8,0, einer Temperatur von 20 °C bis 40 °C und einer Konzentration des Proteins von 0,001 bis 10 Gew.-%, bezogen auf das Gesamtgewicht der für die Bestimmung der Veränderung der Lichtabsorption verwendeten Probenmischung, eine Veränderung der Lichtabsorption von 1 bis 100 % - gemessen wie in der Beschreibung angegeben - bewirkt.

Unter Beeinflussung der Schweißdrüse(n) ist erfindungsgemäß die Beeinflussung der Schweißdrüse(n) dahingehend zu verstehen, dass die Absonderung von Schweiß aus dem Ausführungsgang vermieden bzw. verringert wird. Ohne sich auf eine Theorie beschränken zu wollen kann dies beispielsweise durch Bildung eines Gels und/oder Niederschlags des mindestens einen speziellen Proteins in dem Ausführungsgang der Schweißdrüse bzw. den Ausführungsgängen der Schweißdrüsen geschehen. Weiterhin kann der Einsatz des mindestens einen speziellen Proteins jedoch auch zu einer Störung des Ladungsgleichgewichts innerhalb der Ausführungsgänge der Schweißdrüsen führen. Bezüglich der erfindungsgemäßen Verwendung gilt mutatis mutandis das zu den erfindungsgemäß verwendeten kosmetischen schweißhemmenden Mitteln Gesagte.

Die folgenden Beispiele erläutern die vorliegende Erfindung, ohne sie jedoch darauf einzuschränken:

### Beispiele:

### Formulierungen:

Das in den nachfolgenden Beispielen eingesetzte Protein ist ein hydrolysiertes Protein aus Hülsenfrüchten der Gattung *Pisum,* welches ein mittleres Molekulargewicht M_{w} von etwa 500 Da besitzt:
Erfindungsgemäße schweißhemmende kosmetische Mittel mit einem pH von 2,5 bis 10,0 (Mengenangaben in Gew.-%)

| | 1 | 2 | 3 | 4 | 5 | 6 | 7 |
|---|---|---|---|---|---|---|---|
| Isopropylmyristat | 0,50 | 0,10 | 0,50 | 1,0 | 2,0 | 3,0 | 5,0 |
| Protein | 0,50 | 2,0 | 3,0 | 5,0 | 7,0 | 10 | 20 |
| Eumulgin B3 ^{a)} | 3,0 | 3,0 | 3,0 | 4,0 | 4,0 | 4,0 | 5,0 |
| Parfüm | 0,10 | 0,20 | 0,30 | 0,30 | 0,50 | 0,8 | 1,0 |
| Konservierungsmittel | 0,50 | 0,50 | 0,50 | 0,80 | 0,80 | 1,5 | 2,0 |
| pH-Stellmittel | ad pH | ad pH | ad pH | ad pH | ad pH | ad pH | ad pH |
| Wasser | ad 100 | ad 100 | ad 100 | ad 100 | ad 100 | ad 100 | ad 100 |

| | | | | | | | |
|---|---|---|---|---|---|---|---|
| ^{a)} Eumulgin B3 (INCI: Ceteareth-30; BASF) | | | | | | | |

## Patentansprüche

1. Verwendung einer Kombination, enthaltend
a) mindestens einen Stoff, ausgewählt aus der Gruppe von kosmetischen Ölen, welche bei 20 °C und 1.013 hPa flüssig sind, Riechstoffen und Wachsen,
b) Treibmittel in einer Gesamtmenge von 0 bis 99 Gew.-%, bezogen auf das Gesamtgewicht des schweißhemmenden kosmetischen Mittels, und
c) mindestens ein Protein in einer Gesamtmenge von 0,1 bis 70 Gew.-%, bezogen auf das Gesamtgewicht des schweißhemmenden kosmetischen Mittels, wobei das mindestens eine Protein ein aus Hülsenfrüchten der Gattung *Pisum* isoliertes Protein ist, wobei das Protein hydrolysiert ist und gegebenenfalls kationisch modifiziert ist
und wobei das mindestens eine Protein bei einer pH-Wert Änderung von mindestens 0,5 in einem pH-Bereich von pH 4,0 bis pH 8,0, einer Temperatur von 20 °C bis 40 °C und einer Konzentration des Proteins von 0,001 bis 10 Gew.-%, bezogen auf das Gesamtgewicht der für die Bestimmung der Veränderung der Lichtabsorption verwendeten Probenmischung, eine Veränderung der Lichtabsorption von 1 bis 100 % - gemessen wie in der Beschreibung angegeben - bewirkt
und wobei die Kombination keine Halogenide und/oder Hydroxyhalogenide von Aluminium und/oder Zirconium enthält,
zur Reduzierung und/oder Verhinderung von Schweiß, insbesondere Achselschweiß oder Schweiß anderer Körperregionen.

2. Verwendung nach Anspruch 1, **dadurch gekennzeichnet, dass** das mindestens eine Protein in einer Gesamtmenge von 0,5 bis 60 Gew.-%, vorzugsweise von 1,5 bis 50 Gew.-%, bevorzugt von 2 bis 40 Gew-%, weiter bevorzugt von 2,5 bis 30 Gew.-%, insbesondere von 3 bis 20 Gew.-%, bezogen auf das Gesamtgewicht des schweißhemmenden kosmetischen Mittels, enthalten ist.

3. Verwendung nach einem der Ansprüche 1 oder 2, **dadurch gekennzeichnet, dass** das mindestens eine Protein ein mittleres Molekulargewicht M_{w} von 150 bis 100.000 Da, vorzugsweise von 180 bis 50.000 Da, bevorzugt von 200 bis 10.000 Da, weiter bevorzugt von 250 bis 8.000 Da, insbesondere von 300 bis 5.000 Da, aufweist.

4. Verpackungseinheit (Kit-of-Parts), umfassend - getrennt voneinander konfektioniert -
a) mindestens einen ersten Container (C1), enthaltend ein kosmetisches Mittel (M1) umfassend mindestens einen schweißhemmenden Wirkstoff, und
b) mindestens einen zweiten Container (C2), enthaltend ein kosmetisches Mittel (M2) umfassend mindestens ein Protein, wobei das mindestens eine Protein ein aus Hülsenfrüchten der Gattung *Pisum* isoliertes Protein ist, wobei das Protein hydrolysiert ist und gegebenenfalls kationisch modifiziert ist
und wobei das mindestens eine Protein bei einer pH-Wert Änderung von mindestens 0,5 in einem pH-Bereich von pH 4,0 bis pH 8,0, einer Temperatur von 20 °C bis 40 °C und einer Konzentration des Proteins von 0,001 bis 10 Gew.-%, bezogen auf das Gesamtgewicht der für die Bestimmung der Veränderung der Lichtabsorption verwendeten Probenmischung, eine Veränderung der Lichtabsorption von 1 bis 100 % - gemessen wie in der Beschreibung angegeben - bewirkt
und wobei das kosmetische Mittel (M2) keine Halogenide und/oder Hydroxyhalogenide von Aluminium und/oder Zirconium enthält.

5. Verwendung mindestens eines Proteins zur zumindest teilweisen Beeinflussung der Schweißdrüse(n),
- wobei das mindestens eine Protein ein aus Hülsenfrüchten der Gattung *Pisum* isoliertes Protein ist,
- wobei das Protein hydrolysiert ist und gegebenenfalls kationisch modifiziert ist
- und wobei das mindestens eine Protein bei einer pH-Wert Änderung von mindestens 0,5 in einem pH-Bereich von pH 4,0 bis pH 8,0, einer Temperatur von 20 °C bis 40 °C und einer Konzentration des Proteins von 0,001 bis 10 Gew.-%, bezogen auf das Gesamtgewicht der für die Bestimmung der Veränderung der Lichtabsorption verwendeten Probenmischung, eine Veränderung der Lichtabsorption von 1 bis 100 % - gemessen wie in der Beschreibung angegeben - bewirkt.

## Claims

1. The use of a combination, containing
a) at least one substance selected from the group of cosmetic oils which are liquid at 20 °C and 1,013 hPa, fragrances and waxes,
b) propellants in a total amount of from 0 to 99 wt.%, based on the total weight of the antiperspirant cosmetic agent, and
c) at least one protein in a total amount of from 0.1 to 70 wt.%, based on the total weight of the antiperspirant cosmetic agent, wherein the at least one protein is a protein isolated from legumes of the genus *Pisum,* wherein the protein is hydrolyzed and optionally cationically modified,
and wherein the at least one protein brings about a change in light absorption (measured as specified in the description) from 1 to 100% at a pH change of at least 0.5 in a pH range of from pH 4.0 to pH 8.0, at a temperature of from 20 °C to 40 °C, and at a concentration of the protein of from 0.001 to 10 wt.%, based on the total weight of the sample mixture used for determining the change in light absorption,
and wherein the combination does not contain any halides and/or hydroxyhalides of aluminum and/or zirconium,
for reducing and/or preventing perspiration, in particular axillary perspiration or perspiration at other parts of the body.

2. The use according to claim 1, **characterized in that** the at least one protein is contained in a total amount of from 0.5 to 60 wt.%, preferably from 1.5 to 50 wt.%, more preferably from 2 to 40 wt.%, even more preferably from 2.5 to 30 wt.%, in particular from 3 to 20 wt.%, based on the total weight of the antiperspirant cosmetic agent.

3. The use according to one of claims 1 or 2, **characterized in that** the at least one protein has an average molecular weight M_{w} of from 150 to 100,000 Da, preferably from 180 to 50,000 Da, more preferably from 200 to 10,000 Da, even more preferably from 250 to 8,000 Da, in particular from 300 to 5,000 Da.

4. A packaging unit (kit-of-parts) comprising, packaged separately from one another,
a) at least one first container (C1), containing a cosmetic agent (M1) comprising at least one antiperspirant active ingredient, and
b) at least one second container (C2), containing a cosmetic agent (M2) comprising at least one protein, wherein the at least one protein is a protein isolated from legumes of the genus *Pisum,* wherein the protein is hydrolyzed and optionally cationically modified,
and wherein the at least one protein brings about a change in light absorption (measured as specified in the description) from 1 to 100% at a pH change of at least 0.5 in a pH range of from pH 4.0 to pH 8.0, at a temperature of from 20 °C to 40 °C, and at a concentration of the protein of from 0.001 to 10 wt.%, based on the total weight of the sample mixture used for determining the change in light absorption,
and wherein the cosmetic agent (M2) does not contain any halides and/or hydroxyhalides of aluminum and/or zirconium.

5. The use of at least one protein for at least partially influencing the sweat gland(s),
- wherein the at least one protein is a protein isolated from legumes of the genus *Pisum,*
- wherein the protein is hydrolyzed and optionally cationically modified
- and wherein the at least one protein brings about a change in light absorption (measured as specified in the description) from 1 to 100% at a pH change of at least 0.5 in a pH range of from pH 4.0 to pH 8.0, at a temperature of from 20 °C to 40 °C, and at a concentration of the protein of from 0.001 to 10 wt.%, based on the total weight of the sample mixture used for determining the change in light absorption.

## Revendications

1. Utilisation d'une combinaison, contenant
a) au moins une substance choisie dans le groupe constitué par des huiles cosmétiques liquides à 20 °C et sous 1 013 hPa, des substances odoriférantes et des cires,
b) un agent propulseur en une quantité totale de 0 à 99 % en poids, par rapport au poids total de l'agent cosmétique antisudoral, et
c) au moins une protéine en une quantité totale de 0,1 à 70 % en poids, par rapport au poids total de l'agent cosmétique antisudoral, dans laquelle l'au moins une protéine est une protéine isolée de légumineuses du genre *Pisum,* dans laquelle la protéine est hydrolysée et éventuellement modifiée cationiquement et dans laquelle l'au moins une protéine entraîne une modification de l'absorption de la lumière de l'ordre de 1 à 100 %, mesurée comme indiqué dans la description, suivant une modification de la valeur de pH d'au moins 0,5 dans une plage de pH de 4,0 à 8,0, une température de 20 °C à 40 °C et une concentration de la protéine de 0,001 à 10 % en poids, sur la base du poids total du mélange d'échantillons utilisé pour déterminer la modification de l'absorption de lumière, et dans laquelle la combinaison est exempte d'halogénures et/ou d'hydroxyhalogénures d'aluminium et/ou de zirconium pour réduire et/ou prévenir la transpiration, en particulier la transpiration des aisselles ou la transpiration d'autres parties du corps.

2. Utilisation selon la revendication 1, **caractérisée en ce que** l'au moins une protéine est utilisée est présente en une quantité totale de 0,5 à 60 % en poids, de préférence de 1,5 à 50 % en poids, préférablement de 2 à et 40 % en poids, de manière davantage préférée de 2,5 à 30 % en poids, en particulier de 3 à 20 % en poids, par rapport au poids total de l'agent cosmétique antisudoral.

3. Utilisation selon l'une des revendications 1 ou 2, **caractérisée en ce que** l'au moins une protéine présente un poids moléculaire M_{w} moyen de 150 à 100 000 Da, de préférence de 180 à 50 000 Da, préférablement de 200 à 10 000 Da, de manière davantage préférée de 250 à 8 000 Da, en particulier de 300 à 5 000 Da.

4. Unité de conditionnement (Kits-of-Parts) comprenant, confectionnés séparément les uns des autres,
a) au moins un premier récipient (C1) contenant un agent cosmétique (M1) comprenant au moins une substance active antisudorale, et
b) au moins un second récipient (C2) contenant un agent cosmétique (M2) comprenant au moins une protéine, dans laquelle l'au moins une protéine est une protéine isolée de légumineuses du genre *Pisum,* dans laquelle la protéine est hydrolysée et éventuellement modifiée cationiquement et dans laquelle l'au moins une protéine entraîne une modification de l'absorption de la lumière de 1 à 100 %, mesurée comme indiqué dans la description, suivant une modification de la valeur de pH d'au moins 0,5 dans une plage de pH de 4,0 à 8,0, une température de 20 °C à 40 °C et une concentration de la protéine de 0,001 à 10 % en poids, sur la base du poids total du mélange d'échantillons utilisé pour déterminer la modification de l'absorption de lumière, et dans laquelle l'agent cosmétique (M2) est exempt d'halogénures et/ou d'hydroxyhalogénures d'aluminium et/ou de zirconium.

5. Utilisation d'au moins une protéine pour influencer au moins partiellement la ou les glandes sudoripares,
- dans laquelle l'au moins une protéine est une protéine isolée de légumineuses du genre *Pisum,*
- dans laquelle la protéine est hydrolysée et éventuellement modifiée cationiquement
- et dans laquelle l'au moins une protéine entraîne une modification de l'absorption de la lumière de 1 à 100 %, mesurée comme indiqué dans la description, suivant une modification de la valeur de pH d'au moins 0,5 dans une plage de pH de 4,0 à 8,0, une température de 20 °C à 40 °C et une concentration de la protéine de 0,001 à 10 % en poids, sur la base du poids total du mélange d'échantillons utilisé pour déterminer la modification de l'absorption de lumière.
